# EUROPEAN PATENT APPLICATION

(11) **EP 3 483 896 A1**
(43) Date of publication of application: **15.05.2019**
(21) Application number: 17201127.2
(22) Date of filing: 10.11.2017
(51) Int. Cl.: G16H 40/63

(54) **HUMAN-MACHINE INTERFACE FOR CONTROLLING A MEDICAL DEVICE, METHOD FOR CONTROLLING A MEDICAL IMAGING DEVICE, COMPUTER PROGRAM PRODUCT AND COMPUTER-READABLE MEDIUM**

(71) Applicant: Siemens Healthcare GmbH, 91052 Erlangen (DE)
(72) Inventor: Bauer, Jörg, 90431 Nürnberg (DE); Kuhrt, Sören, 91056 Erlangen (DE); Scholze, Maximilian, 91330 Eggolsheim (DE)

(57) **Abstract**

The present invention suggests a human-machine interface (1) for controlling a medical device (10), in particular a medical imaging device, wherein the human-machine interface (1) has a touch-display (15) and is configured for
- detecting (101) an interaction sequence at the touch display;
- extracting (102) a first parameter (11) and a second parameter (12) from the detected interaction sequence, wherein the first parameter (11) specifies a first part of the interaction sequence and the second parameter (12) specifies a second part of the interaction sequence; and
- transferring (105) a control signal (25) to the medical device (10), when both
-- the first parameter (11) meets a first requirement (21) and
-- the second parameter (12) meets a second requirement (22) .

## Description

The present invention deals with a human-machine interface for controlling a medical device, a method for controlling a medical imaging device, a computer program product and a computer-readable medium.

It is well known from the state of the art to control a medical device, such as a medical imaging device, by a human-machine interface having a touch display. By touching a screen of the touch display at a specific spot, typically a spot on the screen visualising an actuating means, such as a button or a switch, a control signal is triggered for causing a specific operation on the medical device side. In the case of a computer tomography (CT) - device such a specific operation might be related to arranging a table of the CT - device or to start/stop recording a medical imaging data set.

However, these human-machine interfaces can easily trigger the control signal accidently and might therefore causes damages at the patient and/or the medical device. For example, a slashing liquid could contact the touching display at the specific spot of the visualized actuating mean or the patient leans against the touching display and touches the touch display unintended. In another scenario, the touch display has a malfunction and confirms an interaction with the touch display, although the operator or the patient did not contact the touch display. Alternatively, during the signal processing or in the data transfer an error occurs that accidently causes an operation at the medical device.

Considering the above mentioned, it is therefore an object of the present invention to provide a human-machine interface having a reduced probability for triggering an operation at the medical device accidently.

This object is achieved by the human-machine interface for controlling a medical device according to claim 1, by the method for controlling a medical device according to claim 13, the computer program product according to claim 14 and by the computer readable computer medium according to claim 15.

According to a first aspect of the present invention a human-machine interface for controlling a medical device, in particular a medical imaging device, is provided, wherein the human-machine interface has a touch-display and is configured for
- detecting an interaction sequence at the touch display;
- extracting a first parameter and a second parameter from the detected interaction sequence, wherein the first parameter specifies a first part of the interaction sequence and the second parameter specifies a second part of the interaction sequence; and
- transferring a control signal to the medical device, when both
   -- the first parameter meets a first requirement and
   -- the second parameter meets a second requirement.

Contrary to the state of the art, it is provided according to the invention that the interaction sequence for triggering the control signal has to meet both the first requirement and the second requirement. In other words: For triggering the control signal the interaction sequence has to be realized such that in addition to touching the touch display the second parameter can be extracted and need to satisfy the second requirement. In particular, the human-machine interface is configured such that the second part of the interaction sequence confirms the actuating by the first part of the interaction sequence. Thus, a complex interaction sequence having at least a first part and a second part is needed for triggering the control signal. As a consequence, the probability for accidently triggering the control signal is reduced advantageously, since the probability for reproducing the complex interaction sequence unintended is lower than for just contacting the touch display. Another advantage is that by determining the first and second requirement the complexity of the interaction sequence can be defined. Preferably, the human-machine interface is configured such that an user or an operator can select the first requirement and the second requirement, for example from a list of potential requirements, for defining the interaction sequence that needs to be performed for triggering the control signal.

Preferably, the medical device is a medical imaging device, such as a computer tomography (CT)-device. In particular, the control signal is intended for causing an operation at the medical device such as arranging a sample holder, i.e., a patient table, setting parameters for recording a medical imaging data set and/or starting/stopping a record of the medical imaging data set. Thus by transferring the control signal a specific operation can be initiated.

In particular, the touch display is configured such that the touch display has a detection unit that detects a signal depending on the kind of interaction between the operator and the touch display. The skilled person knows that such a detection unit might by based on a resistive system, a capacitive system, an inductive system or the like. Preferably, the human-machine interface is configured such that the first parameter and the second parameter are extracted from the signal detected by the detection unit. For example, the first parameter and/or the second parameter includes information about the location of the contact at the touch display, a duration of the contact and/or a trajectory along touching occurs or occurred. Preferably, the first parameter and/or the second parameter are provided to a control unit, in particular in real time, i.e., the information about the interaction of the first part and the second part are transferred to the control unit during performing the interaction sequence. In particular, the first parameter and/or the second parameter are incorporated into an interaction data set and preferably the interaction data set is transferred to the control unit. By providing the first parameter and the second parameter to the control unit, it is advantageously possible to check subsequently whether the first requirement and the second requirement are meet. Thus, it is for example possible to provide the information that the first requirement has been realized to the operator before the second part of the interaction can be started. Preferably, it is provided that the control unit of the human-machine interface has a processor configured for
- extracting the first parameter and the second parameter from the detected interaction sequence
- comparing the first parameter with the first requirement and comparing the second parameter with the second requirement
- triggering the control signal, when the first parameter meets the first requirement and the second parameter meets the second requirement. Further, it is provided that the control unit comprises a memory device for storing the first requirement and the second requirement.

In general, the term "interaction sequence" describes each interaction between an operator and the touch display. In particular, the operator interacts with the touch display by contacting the touch display by using his finger or a means for contacting the touch display, such as a pen. Further, it is thinkable that during the interaction sequence the contact with the touch screen is interrupted from time to time or the contact remains during the whole interaction sequence.

Particularly advantageous embodiments and features of the invention are given by the dependent claims as revealed in the following description. Features of different claim categories may be combined as appropriate to give further embodiments not described herein.

According to a preferred embodiment, it is provided that for meeting the first requirement and/or the second requirement the first part and/or the second part corresponds to
- touching the touch display, in particular at any spot or at a specific spot,
- touching the touch display along a defined trajectory, in particular along a defined complex trajectory, and/or
- touching the touch display for a defined period of time.
For example the first part is touching the touch display and the second part is touching the touch display along a defined trajectory, for example along a circle, a wave or the like, in particular by starting from the spot at the touch display that is contacted during the first part. Furthermore, it is provided that the touch display visualizes an actuating means, such as a button or a switch, and the first part and/or the second part corresponds to touching the touch display at the spot that visualizes the actuating means. It is also thinkable that touching the touch display corresponds to a wipe or slide movement. Preferably, it is provided that the first part and the second part are identical but are separated in time. In other words, by repeating the first part as second part of the interaction sequence, the second requirement is realized. In another embodiment the first part differs from the second part. Thus, the probability for triggering the control signal accidently is further reduced. In particular, it is provided that the human-machine interface is configured such that an information data set is visualized on the touch display, wherein the information data set informs the operator about the interaction sequence that has to be realized for meeting both the first and the second requirement. For example, the information data set comprises the information about the way of performing the second part of the interaction sequence, in particular after the first part has been finished. For example, the information data set is visualized on the screen by a text passage and/or an animation (arrow, hand and/or the like). Furthermore, it is provided that the operator is informed about the time left for finishing the defined period of time of the second part of the interaction sequence, for example by a progress bar being visualized on the touch screen.

In another embodiment of the present invention, it is provided that the human-machine interface is configured such that the interaction sequence, in particular the second part of the interaction sequence, has to be performed within a defined time interval. In particular, by meeting the first requirement the defined time interval starts. Thus, it is advantageously possible to avoid that the second requirement is met by accident after the first requirement has been met a long time ago. Preferably, the human-machine interface is reset to a status before the interaction sequence, when the defined time interval elapses.

According to another embodiment, it is provided that the human-machine interface is configured such that an error message is generated, when the defined time interval is elapsed without meeting the second requirement by the second parameter. Thus it is advantageously possible to inform the operator or user about a malfunction and/or a misuse of the touch display. Preferably, the touch-display is transferred into a lock mode and/or the medical device is shut down, when the defined time interval is elapsed without meeting the second requirement by the second parameter. For example, an error signal is transferred to the medical devise instead of the control signal.

Preferably, the human-machine interface is configured such that an information about a remaining time for performing the interaction sequence, in particular the second part of the interaction sequence, is provided, in particular at the touch screen. Thus, the operator is informed about the time left for realizing the second requirement by the second part of the interaction sequence. Thereby it is thinkable that the operator is informed by an optic or auditive signal. For example, the colour of the touch display changes during the time interval or a clock or a countdown is visualized on the touch screen.

In particular, it is provided that the human-machine interface is configured such that the first part and the second part are separated in time or at least partially overlap in time. For example, the first part and the second part are separated by interrupting the contact to the touch display. Preferably, the first part and the second part have to be executed subsequently for realizing the first requirement and the second requirement. But it is also thinkable that the first part and the second part have to be realized simultaneously, for example by touching two different spots of the touch display at the same time.

In another embodiment of the present invention, it is provided that the human-machine interface is configured such that a visualisation on the touch-screen is changed between the first part and the second part during the interaction sequence, in particular when the first parameter meets the first requirement. For example, a first panel visualising the actuating means is changed to a second panel visualising a further actuating means that has to be touched for meeting the second requirement.

In particular, it is provided that the human-machine interface is configured such that for meeting the first requirement and/or the second requirement the first part and/or the second part corresponds to touching the touch display along a horizontal line. Thus, it is advantageously avoided that a liquid being splashed on the touch screens and then moves downwards accidently causes an interaction sequence that meet the first and the second embodiment unintended.

Preferably, it is provided that the human-machine interface is configured such that for meeting the first requirement and the second requirement the first part corresponds to touching the touch display at a first spot and the second part corresponds to touching the touch display at a second spot, wherein the first spot and the second spot are separated by a defined distance. Thus, the first requirement and the second requirement can be realized fast, since the first part and the second part can be performed simultaneously.

According to a preferred embodiment it is provided that the distance is greater than 15 cm, preferably more than 15 cm and more preferably more than 20 cm. Due to this comparative large distance it can be avoided that the second spot is touched accidently when the first spot is touched for satisfying the first requirement. Alternatively, it is thinkable that the distance is chosen such that the first spot and the second spot can be reached by two fingers of the same hand. Thus, the touch display can be operated by using only on hand.

Furthermore, it is preferably provided that an interaction data set including the first parameter and/or the second parameter is transferred from the touch-display to a control unit, wherein the control unit is located at the medical imaging device. By transferring the first parameter and the second parameter to the control unit being separated from the touch display it is advantageously possible to avoid errors caused by signal processing at the touch display being unsecured. Thus, the probability for triggering the control signal unintended is further reduced.

Preferably, the human machine device is incorporated into a workstation related to the medical device and/or incorporated into the medical device. A workstation might be a (personal) computer, a virtual running machine on host hardware, a microcontroller, or an integrated circuit. As an alternative, the workstation can be a real or a virtual group of computers. Preferably, the workstation comprises a calculation unit and a memory unit. A calculation unit can comprise hardware elements and software elements, for example a microprocessor or a field programmable gate array. A memory unit can be embodied as non-permanent main memory (e. g. random access memory) or a permanent mass storage (e. g. a hard disk, USB stick, SD card, solid state disk). Preferably, the workstation can be part of the medical imaging device. It is also thinkable that at least one of the steps of the method is performed on a server or at the cloud.

A further aspect of the present invention is a method for controlling a medical imaging device, in particular by using a human-machine interface according to one of the preceding claims, comprising:
- detecting an interaction sequence at the touch display
- extracting a first parameter and a second parameter from the detected interaction sequence, wherein the first parameter and the second parameter respectively specify a part of the interaction sequence, and
- transferring a control signal to the medical device when both the first parameter satisfies a first requirement and the second parameter satisfies a second requirement.

Another aspect of the present invention is a computer program product for carrying out the steps of the method according to the present invention when the computer program product is loaded into a memory of a programmable device.

A further aspect of the present invention is a computer-readable medium on which is stored a program elements that can be read and executed by a computer unit in order to perform steps of the method according to the present invention when the program elements are executed by the computer unit.
- Fig. 1: shows schematically a system comprising a medical imaging device and a human-machine interface according to one preferred embodiment of the present invention,
- Fig.2-8: show different exemplary screen shoots of touch display according to the present invention and
- Fig. 9: shows a flow diagram showing schematically a method for controlling a medical device according to one preferred embodiment of the present invention.

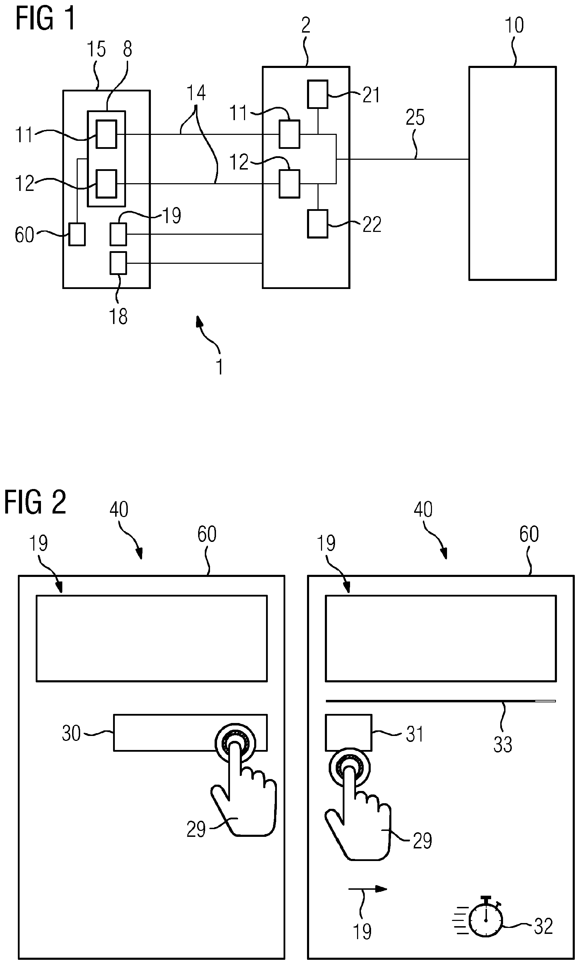

In figure 1 schematically a system 100 comprising a medical device 10 and a human-machine interface 15 according to one preferred embodiment of the present invention is illustrated. For example, the medical device 10 is a medical imaging device such as a computer tomography (CT) device and the human-machine interface 15 is configured for controlling specific operations that can be performed by the medical imaging device. In the case of the computer tomography (CT) device potential operation might be
- transferring a stretcher or table used to arrange a patient relative to a source emitting radiation for recording a medical imaging data set,
- starting and/or stopping the radiation for recording the medical imaging data set and/or
- transferring the medical imaging device between a non-operation mode and an operation mode. In particular, the human-machine interface 1 comprises a touch display 15 that for example visualizes an actuating means 30 on a screen 60 of the touch display 15. According to the state of the art, it is provided that touching the screen 60 of the touch-display, in particular touching the visualized actuating means 30 being visualized on the screen 60, is registered by a detection unit 8 that is incorporated into the touch screen 15. The skilled person knows that such a detection unit 8 might by based on a resistive system, a capacitive system, an inductive system or the like. Preferably, it is provided that a first parameter 11 can be extracted from the signal detected by the detection unit 8, wherein the first parameter 11 specifies the interaction with the touch screen 15. Thereby the first parameter 11 specifies the spot that was touched, for example in form of a point in a coordination system. Preferably, the first parameter 11 is incorporated into an interaction data set 14, wherein the interaction data set 14 is transferred to control unit 2. The control unit 2 is configured such that a control signal 25 for triggering a specific operation at the medical device 20 is transferred to the medical device 20, when the first parameter 11 meets a first requirement 21. For example, the first requirement 12 is touching the screen 60 of the touch display 15 at a defined spot, in particular at the spot of the screen 60 that visualizes the actuating means 8. By comparing the first parameter 11 to the first requirement 21, it is advantageously possible to check whether the touch display 15 has been used in such a way that the control signal 25 for triggering the operation at the medical device 10 can be transferred.

However, there are scenarios in that the control signal 25 is generated accidently or wrongly. For example, the touch display 15 has a malfunction and produces an interaction data 14 signal that generates a control signal 25 or the screen 60 of the touch display 15 is contacted accidently by a splashing liquid or by the operator or by a stumbling patient. It is also thinkable that there is a malfunction that affects the transfer of the interaction data set 14 to the control unit 2.

As a consequence of generating the control signal 25 accidently or wrongly, an operation of the medical device 10 is caused unintended, in particular in such a way that a patient or the medical device 10 might be endangered.

For avoiding that the control signal 25 is generated by accident, the human-machine interface 1 is configured for
- detecting an interaction sequence at the touch display 15
- extracting a first parameter 11 and a second parameter 12 from the detected interaction sequence, wherein the first parameter 11 specifies a first part of the interaction sequence and the second parameter 12 specifies a second part of the interaction sequence, and
- transferring a control signal 25 to the medical device when both the first parameter 11 satisfies a first requirement 21 and the second parameter 12 satisfies a second requirement 22. As a result, the control signal 25 is emitted only in such cases in that in addition to the first requirement 21 the second requirement 22 is met. Preferably, it is provided that the human-machine interface 1 is configured such that the second part of the interaction sequence confirms the first part of the interaction sequence. In particular, the second parameter 12 confirms meeting the first requirement 21 by the first parameter 11 by choosing the interaction sequence having both the proper first part and the proper second part. Thus, the probability for emitting the control signal 25 accidently is reduced, advantageously. In particular, the first part and the second part are different or performed subsequently and thus the first parameter 11 and the second parameter 12 can be separated from each other unambiguously. It is also thinkable that an error massage 18 is generated, when the second parameter 12 meets the second requirement 22 within a defined time interval, and/or an information data set 19 is generated, when the first requirement is met. The information data set 19 preferably comprises information about the second part of the interaction data set and might be visualized on the screen by an illustration, such as an arrow or a hand, or by an illustrated text passage.

By using an interaction sequence having a first part and a second part, it is possible to define for example a complex interaction sequence that has to be realized for triggering the emission of the control signal to the medical device.

The figures 2 to 8 show examples for interaction sequences of different embodiments according to the present invention. Thereby, a hand 29 of the operator is illustrated in the figures 2 to 8. For example, figure 2 shows on the left side a visualisation of screen 60, wherein an actuating means 30 in form of a button is illustrated. The first requirement 21 is assigned to touching this button by using the hand 29 or by using one finger of the hand 29. By touching the button the visualization changes and a sliding element 31 is illustrated on the display 60 (see right side of figure 2). For meeting the second requirement 22 the slide element 31 has to be slid from the left side to the right side, in particular within a defined time interval. I.e. the interaction sequence comprises a first part and a second part, wherein the first part is specified by the first parameter 11 and the second part is specified by the second parameter 12. In particular, the first part and the second part have to be realized subsequently. Preferably, it is provided that the operator is informed about the time left for realizing the second requirement. In figure 2 a clock 32 being illustrated on the screen 60 represents this visualisation for informing the operator about the time left for realizing the second requirement. Furthermore, it is provided that an information data set 19 is provided to the operator to inform the user how he can realizes the interaction sequence that generates a first parameter 11 and a second parameter 12 for meeting both the first requirement 21 and the second requirement 22. In the embodiment the information data set 19 is visualized both as a text passage during the whole interaction sequence and as an animation (arrow, hand) visualized when the first requirement 21 is met. Preferably, the information data set 19 is sent to the touch display 15 after the first requirement 21 has been realized by the first parameter 11.

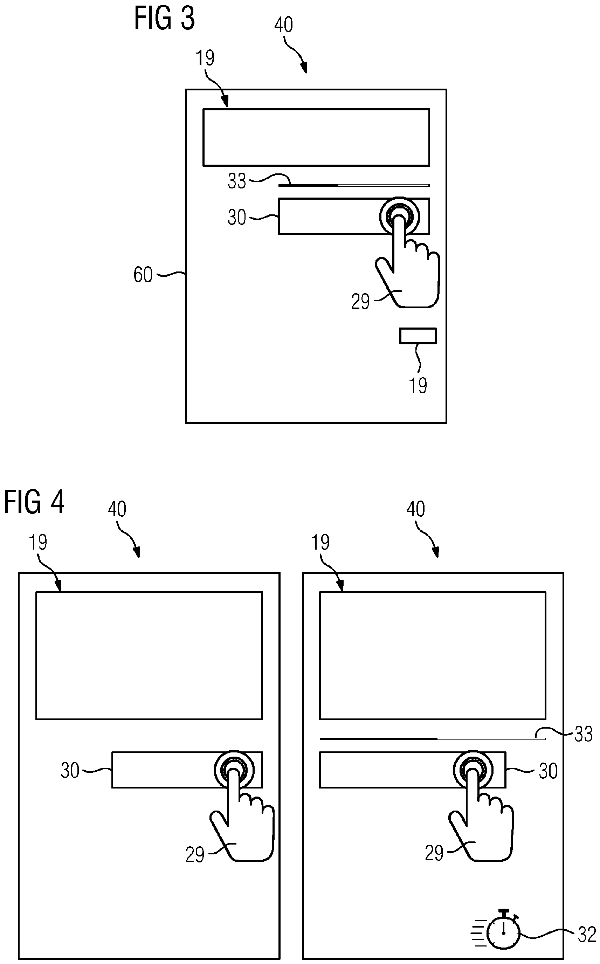

In figure 3 the first requirement 21 is realized by touching the button, i.e. the actuating means 30, and the second requirement 22 is assigned to a period of time in which touching the screen 60 is maintained. In contrast to the embodiment of figure 2, the first part and the second part of the interaction sequence are performed simultaneously for at least a short time. For informing the operator about the time left for realizing the second requirement 21 a progress bar 33 is visualized on the screen 60 of the touch display 15.

In figure 4 the first requirement 21 and the second requirement 22 are realized by the same interaction with the screen 60 wherein the interactions are shifted in time. In particular, the first requirement 21 and the second requirement 22 are touching the screen 60 at the same spot subsequently within a defined time interval. Preferably, the progress bar 33 visualizes the time left for touching the same button for the second time.

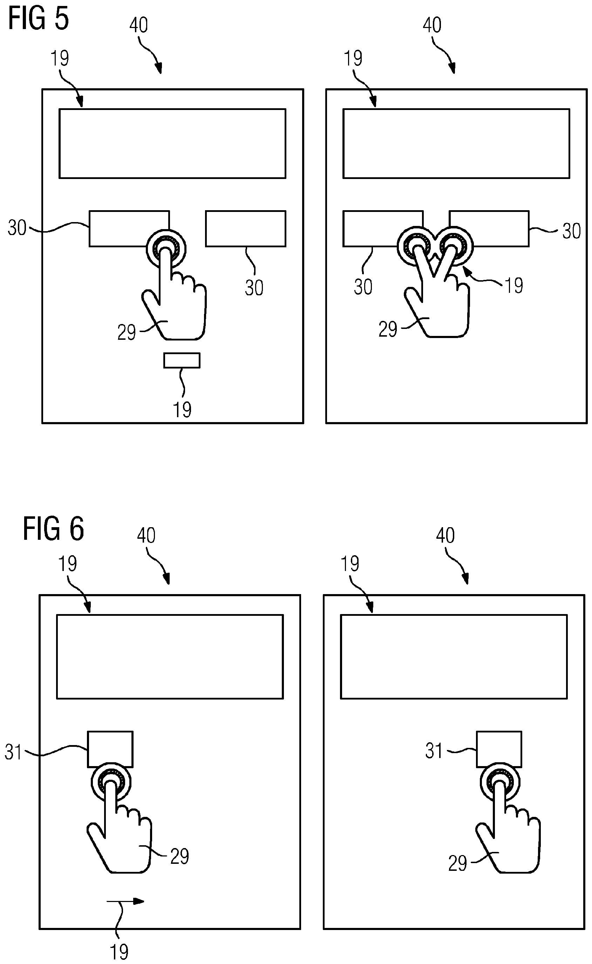

In figure 5 the first requirement 21 is touching the button on the left side of the screen 60 and the second requirement 22 is touching the button on the right side of the screen 60 and the button on the left side of the screen 60 simultaneously. For supporting the operator, the button on the right side is highlighted as soon as the first requirement 21 is met by the first parameter 11.

In figure 6 the first requirement is touching the screen 60, in particular at the spot visualizing the sliding element 31, and the second requirement 22 is touching the display along a trajectory, in particular a horizontal line.

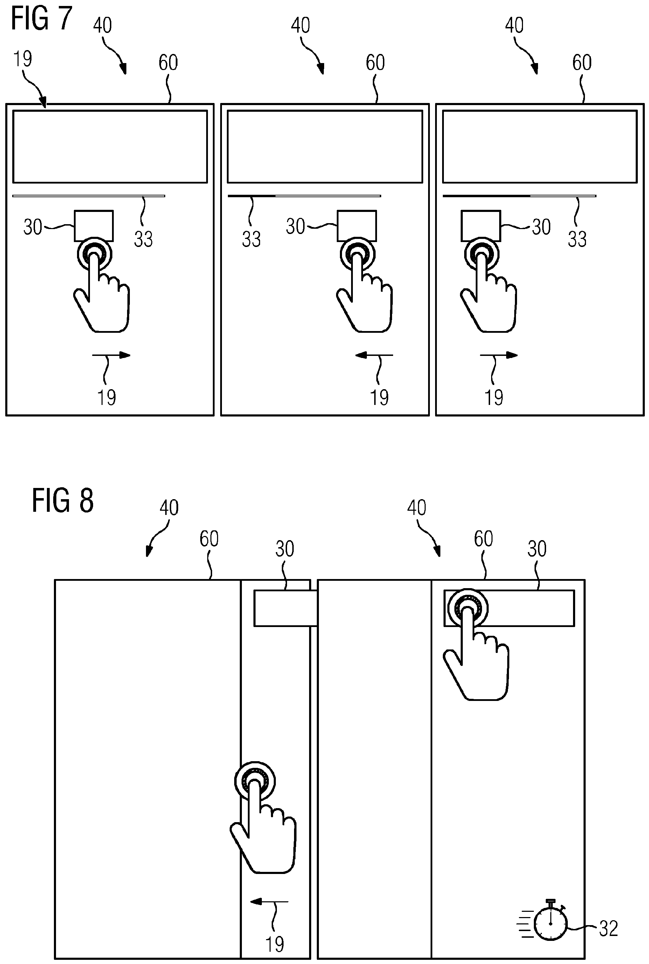

In figure 7 the first requirement 21 is touching the screen 60 of the touch display 15, in particular at the spot visualizing the sliding element 31, and the second requirement is touching the screen back and forth along a horizontal line.

In figure 8 the first requirement is touching the touch display 15 at the edge of the screen 60 of the touch display 15, in particular, by a wipe movement of the finger during the touching, and the second requirement 22 is touching the button that is visualized by satisfying the first requirement 21.

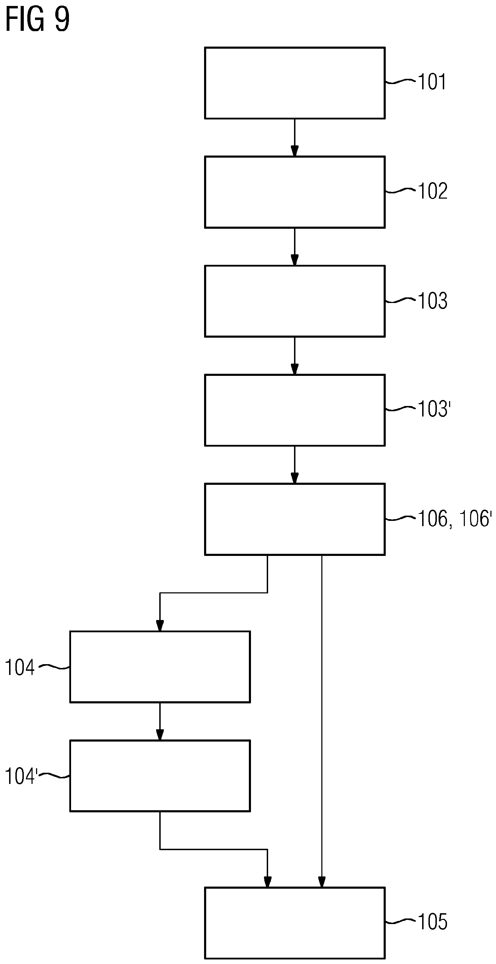

In figure 9 a flow diagram showing schematically a method for controlling a medical device 10 according to one preferred embodiment of the present invention is illustrated. Preferably, it is provided that the method comprises
- detecting 101 an interaction sequence at the touch display 15; and/or
- extracting 102 a first parameter 11 and a second parameter 12 from the detected interaction sequence, wherein the first parameter 11 specifies a first part of the interaction sequence and the second parameter 12 specifies a second part of the interaction sequence;
- generating 103 an interaction data set 14 comprising information about the interaction sequence being detected by the detection unit 8; and/or
- transferring 103' the interaction data set 14 to a control unit 2; and/or
- comparing 106 the first parameter 11 with the first requirement 21 and comparing 106' the second parameter 12 with the second requirement 22; and/or
- generating 104 an error message 18 and/or an information data set 19 and/or
- transferring 104' the error message 18 and/or the information data set 19 to the touch display 15; and/or
- transferring 105 a control signal 25 to the medical device 10 when both the first parameter 11 meets a first requirement 21 and the second parameter 12 meets a second requirement 22.

## Claims

1. Human-machine interface (1) for controlling a medical device (10), in particular a medical imaging device, wherein the human-machine interface (1) has a touch-display (15) and is configured for
- detecting (101) an interaction sequence at the touch display;
- extracting (102) a first parameter (11) and a second parameter (12) from the detected interaction sequence, wherein the first parameter (11) specifies a first part of the interaction sequence and the second parameter (12) specifies a second part of the interaction sequence; and
- transferring (105) a control signal (25) to the medical device (10), when both
-- the first parameter (11) meets a first requirement (21) and
-- the second parameter (12) meets a second requirement (22) .

2. Human-machine interface (1) according to claim 1, wherein for meeting the first requirement (21) and/or the second requirement (22) the first part and/or the second part corresponds to
- touching the touch display (15), for example at any spot or at a specific spot,
- touching the touch display (15) along a defined trajectory, in particular along a defined complex trajectory, and/or
- touching the touch display (15) for a defined period of time.

3. Human-machine interface (1) according to one of the preceding claims, wherein the human-machine interface (1) is configured such that the interaction sequence, in particular the second part of the interaction sequence, has to be performed within a defined time interval.

4. Human-machine interface (1) according to claim 3, wherein the human-machine interface (1) is configured such that an error message (18) is generated, when the defined time interval is elapsed without meeting the second requirement (22) by the second parameter (12).

5. Human-machine interface (1) according to claim 3 or 4, wherein the human-machine interface (1) is configured such that an information about a remaining time for performing the interaction sequence, in particular the second part of the interaction sequence, is provided, in particular at the touch screen (15).

6. Human-machine interface (1) according to one of the preceding claims, wherein the first part and the second part are separated in time or at least partially overlap in time.

7. Human-machine interface (1) according to one of the preceding claims, wherein the human-machine interface (1) is configured such that a visualisation on the touch-screen (15) is changed between the first part and the second part during the interaction sequence, in particular when the first parameter (11) meets the first requirement (21).

8. Human-machine interface (1) according to one of the preceding claims, wherein the human-machine interface (1) is configured such that for meeting the first requirement (21) and/or the second requirement (22) the first part and/or the second part corresponds to touching the touch display (15) along a horizontal line.

9. Human-machine interface (1) according to one of the preceding claims, wherein the human-machine interface (1) is configured such that for meeting the first requirement (21) and/or the second requirement (22) the first part corresponds to touching the touch display (15) at a first spot and the second part corresponds to touching the touch display (15) at a second spot, wherein the first spot and the second spot are separated by a defined distance.

10. Human-machine interface (1) according to claim 8, wherein the distance is greater than 15 cm, preferably more than 15 cm and more preferably more than 20 cm.

11. Human-machine interface (1) according to one of the preceding claims, wherein an interaction data set (14) including the first parameter (11) and/or the second parameter (12) is transferred from the touch-display (15) to a control unit (2), wherein the control unit (2) is located at the medical device (10).

12. Human machine device (1) according to one of the preceding claims, wherein the human machine device (1) is incorporated into a workstation related to the medical device (10) and/or incorporated into the medical device (10) .

13. Method for controlling a medical device (10), in particular by using a human-machine interface (1) according to one of the preceding claims, comprising:
- detecting (101) an interaction sequence at the touch display (15);
- extracting (102) a first parameter (11) and a second parameter (12) from the detected interaction sequence, wherein the first parameter (11) and the second parameter (12) respectively specify a part of the interaction sequence, and
- transferring (105) a control signal (25) to the medical device (10) when both the first parameter (11) satisfies a first requirement (21) and the second parameter (12) satisfies a second requirement (22).

14. Computer program product for carrying out the steps of the method according to any of claims 1 to 12 when the computer program product is loaded into a memory of a programmable device.

15. A computer-readable medium on which is stored a program elements that can be read and executed by a computer unit in order to perform steps of the method according to any of the claims 1 to 12 when the program elements are executed by the computer unit

## Amended claims

### Amended claims in accordance with Rule 137(2) EPC.

1. Human-machine interface (1) for controlling a medical device (10), wherein the medical device is a medical imaging device, wherein the human-machine interface (1) has a touch-display (15) and is configured for
- detecting (101) an interaction sequence at the touch display;
- extracting (102) a first parameter (11) and a second parameter (12) from the detected interaction sequence, wherein the first parameter (11) specifies a first part of the interaction sequence and the second parameter (12) specifies a second part of the interaction sequence; and
- transferring (105) a control signal (25) to the medical device (10), when both
-- the first parameter (11) meets a first requirement (21) and
-- the second parameter (12) meets a second requirement (22) by providing the first parameter (11) and the second parameter (12) to a control unit for checking whether the first requirement (21) and the second requirements (22) are met,
wherein for meeting the first requirement (21) and/or the second requirement (22) the first part and/or the second part corresponds to
- touching the touch display (15) along a defined trajectory, in particular along a defined complex trajectory, and/or
- touching the touch display (15) for a defined period of time.

2. Human-machine interface (1) according to one of the preceding claims, wherein the human-machine interface (1) is configured such that the interaction sequence, in particular the second part of the interaction sequence, has to be performed within a defined time interval.

3. Human-machine interface (1) according to claim 2, wherein the human-machine interface (1) is configured such that an error message (18) is generated, when the defined time interval is elapsed without meeting the second requirement (22) by the second parameter (12).

4. Human-machine interface (1) according to claim 2 or 3, wherein the human-machine interface (1) is configured such that an information about a remaining time for performing the interaction sequence, in particular the second part of the interaction sequence, is provided, in particular at the touch screen (15).

5. Human-machine interface (1) according to one of the preceding claims, wherein the first part and the second part are separated in time or at least partially overlap in time.

6. Human-machine interface (1) according to one of the preceding claims, wherein the human-machine interface (1) is configured such that a visualisation on the touch-screen (15) is changed between the first part and the second part during the interaction sequence, in particular when the first parameter (11) meets the first requirement (21).

7. Human-machine interface (1) according to one of the preceding claims, wherein the human-machine interface (1) is configured such that for meeting the first requirement (21) and/or the second requirement (22) the first part and/or the second part corresponds to touching the touch display (15) along a horizontal line.

8. Human-machine interface (1) according to one of the preceding claims, wherein the human-machine interface (1) is configured such that for meeting the first requirement (21) and/or the second requirement (22) the first part corresponds to touching the touch display (15) at a first spot and the second part corresponds to touching the touch display (15) at a second spot, wherein the first spot and the second spot are separated by a defined distance.

9. Human-machine interface (1) according to claim 7, wherein the distance is greater than 15 cm, preferably more than 15 cm and more preferably more than 20 cm.

10. Human-machine interface (1) according to one of the preceding claims, wherein an interaction data set (14) including the first parameter (11) and/or the second parameter (12) is transferred from the touch-display (15) to a control unit (2), wherein the control unit (2) is located at the medical device (10).

11. Human machine device (1) according to one of the preceding claims, wherein the human machine device (1) is incorporated into a workstation related to the medical device (10) and/or incorporated into the medical device (10) .

12. Method for controlling a medical device (10) by using a human-machine interface (1) according to one of the preceding claims, comprising:
- detecting (101) an interaction sequence at the touch display (15);
- extracting (102) a first parameter (11) and a second parameter (12) from the detected interaction sequence, wherein the first parameter (11) and the second parameter (12) respectively specify a part of the interaction sequence, and
- transferring (105) a control signal (25) to the medical device (10) when both the first parameter (11) satisfies a first requirement (21) and the second parameter (12) satisfies a second requirement (22) by providing the first parameter (11) and the second parameter (12) to a control unit for checking whether the first requirement (21) and the second requirement (22) are met, wherein for meeting the first requirement (21) and/or the second requirement (22) the first part and/or the second part corresponds to
- touching the touch display (15) along a defined trajectory, in particular along a defined complex trajectory, and/or
- touching the touch display (15) for a defined period of time.

13. Computer program product for carrying out the steps of the method according to any of claims 1 to 11 when the computer program product is loaded into a memory of a programmable device.

14. A computer-readable medium on which is stored a program elements that can be read and executed by a computer unit in order to perform steps of the method according to any of the claims 1 to 11 when the program elements are executed by the computer unit
